# EUROPÄISCHE PATENTSCHRIFT

(11) **EP 2 387 369 B1**
(45) Veröffentlichungstag und Bekanntmachung des Hinweises auf die Patenterteilung: **03.09.2014**
(21) Anmeldenummer: 10739292.0
(22) Anmeldetag: 21.06.2010
(51) Int. Cl.: A61B 19/00, F21K 99/00

(54) **MEDIZINISCHES HANDGERÄT ZUR BELEUCHTUNG**
HANDHELD MEDICAL DEVICE FOR ILLUMINATION
DISPOSITIF MÉDICAL PNEUMATIQUE MANUEL POUR ILLUMINATION

(30) Priorität: 22.06.2009 DE 102009030060
(43) Veröffentlichungstag der Anmeldung: 23.11.2011
(73) Patentinhaber: Geuder AG, 69126 Heidelberg (DE)
(72) Erfinder: GEUDER, Volker, 69126 Heidelberg (DE); DRAHEIM, René, 69207 Sandhausen (DE); MÜLLER, Volker, 69120 Heidelberg (DE)
(74) Vertreter: Ullrich & Naumann
(86) Internationale Anmeldenummer: PCT/DE2010/000697
(87) Internationale Veröffentlichungsnummer: WO 2010/149136

(56) Entgegenhaltungen:
- WO-A2-03/021329
- DE-A1-102006 051 736

## Beschreibung

Die Erfindung betrifft ein medizinisches Handgerät zur Beleuchtung, insbesondere zur Anwendung in der Ophthalmologie, mit einem als Griffstück dienenden Gehäuse, einer sich vom Gehäuse aus erstreckenden Sonde mit einem darin verlaufenden Lichtleiter und einem endseitigen Lichtauslass, und einer in das Gehäuse integrierten Lichtquelle.

Medizinische Handgeräte zur Beleuchtung sind aus der Praxis in den unterschiedlichsten Ausführungsformen bekannt. Sofern es sich dabei um Handgeräte zur Anwendung in der Ophthalmologie handelt, ist regelmäßig eine äußerst kleine Bauweise erforderlich. Dies gilt insbesondere für die sich vom Gehäuse aus ersteckende Sonde, die endseitig einen Lichtauslass aufweist. Sofern die Sonde zum Einführen in den menschlichen Körper dient, beispielsweise in das menschliche Auge, ist neben der kleinen Bauweise auch eine ungehinderte Handhabung des Instruments erforderlich.

Gattungsbildende medizinische Handgeräte, insbesondere zur Anwendung in der Ophthalmologie, sind bspw. aus der DE 10 2006 051 736 A1 bekannt. Sie bauen äußerst klein und umfassen im Handgriff eine LED-Lichtquelle. Gespeist werden solche Handgeräte entweder über eine im Handgriff befindliche Batterie oder über einen nach außen führenden Stromanschluss, der regelmäßig mit einer Steuereinheit verbunden ist.

Wenngleich LEDs heutzutage eine ganz erhebliche Lichtstärke aufweisen, ist diese noch lange nicht vergleichbar mit herkömmlichen Leuchtmitteln wie bspw. HalogenLampen. Soll die Leuchtdiode zum Einkoppeln von Licht in einen Lichtleiter, bspw. in eine Glasfaser, dienen, treten erhebliche Verluste auf, zumal die LED das Licht extrem streut und nur ein geringer Teil des emittierten Lichts tatsächlich zur Einkopplung in die Glasfaser zur Verfügung steht. Dies macht den Einsatz von LEDs in gattungsbildenden medizinischen Handgeräten problematisch, wenngleich die damit verbundene Miniaturisierung erhebliche Vorteile mit sich bringt.

WO 03/021329 offenbart die Merkmale des Oberbegriffs des Anspruchs 1.

Der vorliegenden Erfindung liegt daher die Aufgabe zugrunde, ein medizinisches Handgerät zur Beleuchtung, insbesondere zur Anwendung in der Ophthalmologie, derart auszugestalten und weiterzubilden, dass unter Nutzung einer geeigneten LED-Lichtquelle mit Konstruktür einfachen Mitteln eine optimale Lichtausbeute und somit eine hinreichend gute Beleuchtung unter Nutzung einer einen Lichtleiter umfassenden Sonde möglich ist.

Die voranstehende Aufgabe wird erfindungsgemäß durch ein medizinisches Handgerät mit den Merkmalen des Patentanspruchs 1 gelöst. Danach ist das gattungsbildende medizinische Handgerät dadurch gekennzeichnet, dass die Lichtquelle als LED-Lichtquelle mit einer Licht emittierenden Fläche ausgeführt ist und dass der Lichtleiter unmittelbar an die Licht emittierende Fläche angrenzt.

Es hat sich herausgestellt, dass sich zum Einkoppeln in einen Lichtleiter LED-Lichtquellen mit einer Licht emittierenden Fläche ganz besonders eignen, nämlich dann, wenn der Lichtleiter unmittelbar an die Licht emittierende Fläche angrenzt. Dabei werden nicht etwa LEDs mit sogenannten Bonddrähten in einem sonst üblichen transparenten Abdeckkörper verwendet. Vielmehr geht es hier um den Einsatz von LED-Lichtquellen, die eine Licht emittierende Fläche umfassen, die üblicher Weise mit einer Art Silikonlinse bzw. mit einer Abdeckmaske überzogen ist. Solche LED-Lichtquellen streuen das Licht nahezu um 180°.

Eine solche LED-Lichtquelle wird - ohne den Abdeckkörper - in idealer Weise zur Einkopplung von Licht in einen Lichtleiter verwendet, nämlich dann, wenn der Lichtleiter unmittelbar an die Licht emittierende Fläche angrenzt, wenn man nämlich den Lichtleiter bündig an die Fläche heranführt. Entsprechend wird das Licht nicht etwa über die sonst übliche Linse gestreut, gelangt vielmehr unmittelbar von der Licht emittierenden Fläche in den Lichtleiter hinein, so dass eine maximale/optimale Lichtausbeute realisierbar ist.

In vorteilhafter Weise handelt es sich bei der LED-Lichtquelle um einen sogenannten Leuchtchip, wie er bspw. aus der DE 20 2006 018 846 U1 - für sich gesehen - bekannt ist. Wesentlich ist dabei, dass bei dem Leuchtchip eine Licht emittierende Fläche vorgesehen ist, die - bei entsprechender Ausgestaltung des Leuchtchips - entsprechend der Ausgestaltung des Querschnitts einer Lichtleitfaser als runde Fläche - ausgeführt sein kann. Eine Anpassung der Licht emittierenden Fläche an die Querschnittsfläche des Lichtleiters ist von Vorteil.

Bei dem Lichtleiter kann es sich um eine klassische Lichtleitfaser handeln, vorzugsweise um eine Glasfaser, die sich vom Handgriff des medizinischen Handgeräts durch die Sonde hindurch bis zum offenen Ende der Sonde erstreckt. Dort ist der Lichtaustritt vorgesehen.

Im Konkreten kann es sich bei dem Lichtleiter um eine Monofaser oder aber auch um ein Faserbündel handeln, je nach Bedarf. Die die Lichtleitfaser beinhaltende Sonde kann geradlinig oder aber auch gebogen ausgeführt sein. Darin ist ein besonderer Vorteil der Lichtleitfaser zu sehen, so dass nahezu beliebige Ausgestaltungen der Sonde denkbar sind.

Zur optimalen Auskopplung des Lichts in den Lichtleiter ist es von weiterem Vorteil, wenn der Lichtleiter in etwa orthogonal zu der Licht emittierenden Fläche ausgerichtet ist. Grundsätzlich ist es denkbar, dass der Lichtleiter in seiner Position zu der Licht emittierenden Fläche durch entsprechende Halte-/Positionierungs-/Justagemittel gehalten ist.

Zur besonders einfachen Ausgestaltung der Positionierung des Lichtleiters zur Licht emittierenden Fläche der LED-Lichtquelle ist es von Vorteil, wenn der Lichtleiter des im Anspruch 1 definierten Handgeräts endseitig mit der Licht emittierenden Fläche verklebt ist. Vorzugsweise ist der Lichtleiter mit der Licht emittierenden Fläche bündig verklebt. Bei dem Klebstoff handelt es sich um einen transparenten Klebstoff, der im Bereich der dort entstehenden Wärme temperatur- und alterungsbeständig ist. Eine Anpassung der thermischen Ausdehnungskoeffizienten der unterschiedlichen Materialien ist ebenfalls von Vorteil.

Im Konkreten ist es denkbar, dass der Klebstoff die Licht emittierende Fläche insgesamt im Sinne einer Abdeckmaske überdeckt, wobei der Lichtleiter zur Kontaktierung mit der Licht emittierenden Fläche in den Klebstoff hineingetaucht wird, und zwar vorzugsweise bis zur vollständigen Kontaktierung der Licht emittierenden Fläche.

In erfindungsgemäßer Weise ist der über die Querschnittsfläche des Lichtleiters hinausragende Bereich durch eine lichtundurchlässige Abdeckmaske überdeckt, so dass das aus der Fläche heraus emittierte Licht komplett in den Lichtleiter eingekoppelt wird. Ergänzend kann die Licht emittierende Fläche in etwa dem Faserquerschnitt angepasst sein.

Schließlich ist es von weiterem Vorteil, wenn der Verbindungsbereich zwischen der Lichtleitfaser und der Licht emittierender Fläche optisch und/oder mechanisch versiegelt ist. Unter einer mechanischen Versiegelung ist eine Stabilisierung der Verbindung zu verstehen. Eine optische Versiegelung dient zur Abschottung der Lichtquelle nach außen, so dass das gesamte Licht zur Einkopplung in den Lichtleiter zur Verfügung steht. Die Versiegelung kann im Sinne eines Überzugs hergestellt sein, der die Lichtquelle insgesamt und den Endbereich des Lichtleiters überdeckt und somit auch die Verbindung zwischen der LED-Lichtquelle und dem Lichtleiter begünstigt bzw. stabilisiert.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigt
- Fig. 1: in einer schematischen Ansicht ein Ausführungsbeispiel einer Lichtquelle mit Lichteinkopplung in einen Lichtleiter zur Verwendung in einem erfindungsgemäßen medizinischen Handgerät und
- Fig. 2: die verwendete LED-Lichtquelle in handelsüblicher Form.

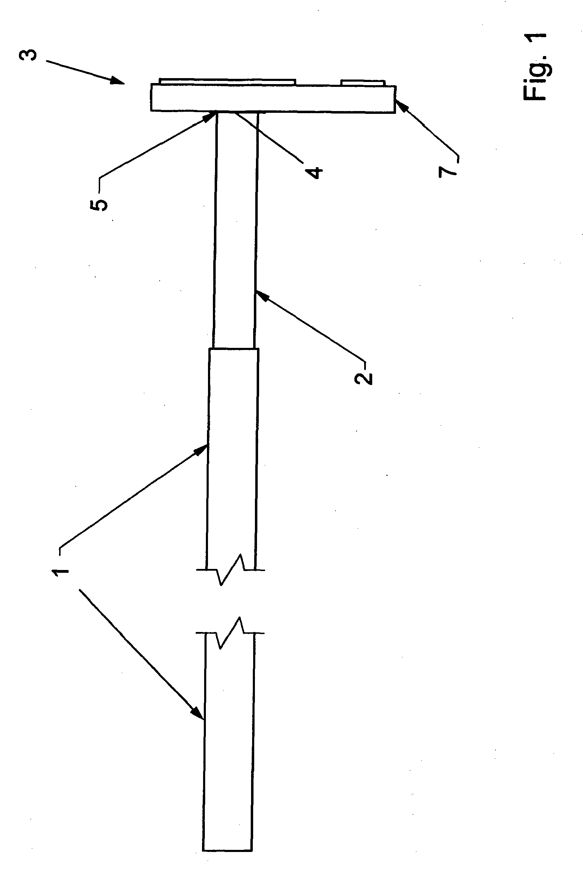

Fig. 1 zeigt einen Teil des "Innenlebens" eines medizinischen Handgeräts zur Beleuchtung, insbesondere zur Anwendung in der Ophthalmologie, wobei ein solches Handgerät ein in der Figur nicht gezeigtes Gehäuse umfasst, welches üblicher Weise als Griffstück dient. Von dem Gehäuse aus erstreckt sich eine Sonde 1 mit einem darin verlaufenden Lichtleiter 2. Bei dem Lichtleiter 2 handelt es sich bspw., jedoch nicht zwingend, um eine Monofaser aus Glas.

Des Weiteren ist eine in das nicht gezeigte Gehäuse integrierte LED-Lichtquelle 3 vorgesehen, deren Licht in den Lichtleiter 2 eingekoppelt wird. Einer einfachen Darstellung halber sind die elektrischen Anschlüsse der LED-Lichtquelle sowie die Stromversorgung nicht gezeigt, zumal es bei der beanspruchten Lehre darauf nicht ankommt.

Bei dem Lichtleiter 2 kann es sich grundsätzlich auch um ein Faserbündel handeln. Bei der Verwendung von Monofasern sind beim Einsatz in der Ophthalmologie Faserquerschnitte von 20 bis 25 Gage von Vorteil.

Fig. 1 lässt des Weiteren erkennen, dass die LED-Lichtquelle 3 eine Licht emittierende Fläche 4 aufweist. Im Bereich der Licht emittierenden Fläche 4 ist der Lichtleiter 2, genauer gesagt die Monofaser, an die Licht emittierende Fläche angekoppelt, so dass das emittierte Licht der LED-Lichtquelle 3 unmittelbar in den Lichtleiter 2 eingespeist wird.

Die Verbindung zwischen der LED-Lichtquelle 3 bzw. der Licht emittierenden Fläche 4 und dem Lichtleiter 2 ist durch einen transparenten Klebstoff 5 bewerkstelligt.

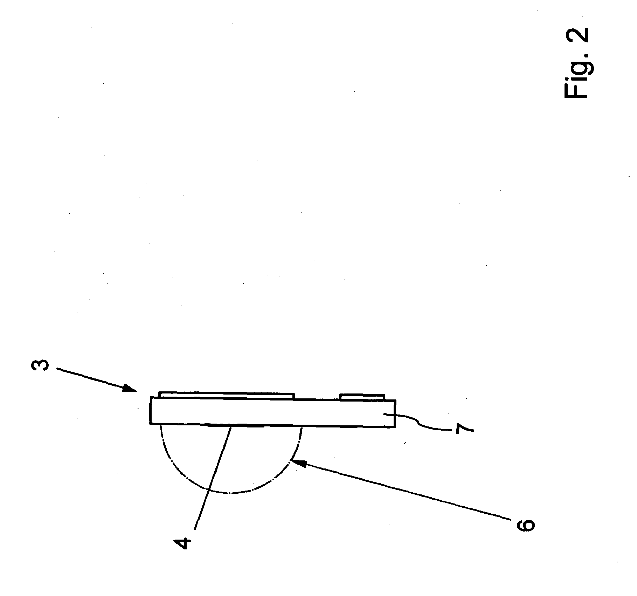

Fig. 2 zeigt die in Fig. 1 verwendete LED-Lichtquelle 3 in einem marktüblichen Zustand, wonach die Licht emittierende Fläche 4 durch eine transparente Linse 6, als Abdeckmaske bezeichnet, überdeckt ist. Entsprechend der Ausgestaltung der Linse wird das Licht in einem Bereich von nahezu 180° zum Substrat 7 bzw. Träger der LED-Lichtquelle 3 emittiert.

Schließlich sei angemerkt, dass das voranstehend erörterte Ausführungsbeispiel lediglich der beispielhaften Erörterung des erfindungsgemäßen medizinischen Handgeräts dient, dieses jedoch nicht auf das Ausführungsbeispiel einschränkt.

### Bezugszeichenliste

- 1: Sonde
- 2: Lichtleiter
- 3: LED-Lichtquelle
- 4: Licht emittierende Fläche (von 3)
- 5: Klebstoff
- 6: Linse
- 7: Substrat

## Patentansprüche

1. Medizinisches Handgerät zur Beleuchtung, insbesondere zur Anwendung in der Ophthalmologie, mit einem als Griffstück dienenden Gehäuse, einer sich vom Gehäuse aus erstreckenden Sonde (1) mit einem darin verlaufenden Lichtleiter (2) und einem endseitigen Lichtauslass, und einer in das Gehäuse integrierten Lichtquelle (3),
wobei die Lichtquelle (3) als LED-Lichtquelle mit einer Licht emittierenden Fläche (4) ausgeführt ist, wobei der Lichtleiter (2) unmittelbar an die Licht emittierende Fläche (4) angrenzt und wobei der Lichtleiter (2) endseitig mit der Licht emittierenden Fläche (4) verklebt ist,
**dadurch gekennzeichnet, dass** der über die Querschnittsfläche des Lichtleiters (2) hinausragende Bereich der Licht emittierenden Fläche durch eine lichtundurchlässige Abdeckmaske überdeckt ist, so dass das aus der Fläche heraus emittierte Licht komplett in den Lichtleiter eingekoppelt wird.

2. Handgerät nach Anspruch 1, **dadurch gekennzeichnet, dass** die Lichtquelle (3) als Leuchtchip ausgeführt ist.

3. Handgerät nach Anspruch 1 oder 2, **dadurch gekennzeichnet, dass** der Lichtleiter (2) als Lichtleitfaser, vorzugsweise als Glasfaser, ausgeführt ist.

4. Handgerät nach Anspruch 3, **dadurch gekennzeichnet, dass** der Lichtleiter (2) als Monofaser ausgeführt ist.

5. Handgerät nach Anspruch 3, **dadurch gekennzeichnet, dass** der Lichtleiter (2) als Faserbündel ausgeführt ist.

6. Handgerät nach einem der Ansprüche 1 bis 5, **dadurch gekennzeichnet, dass** der Lichtleiter (2) in etwa orthogonal zu der Licht emittierenden Fläche (4) ausgerichtet ist.

7. Handgerät nach einem der Ansprüche 1 bis 6, **dadurch gekennzeichnet, dass** der Lichtleiter (2) mit mindestens einer planen Fläche an die Licht emittierende Fläche (4) angrenzt.

8. Handgerät nach einem der Ansprüche 1 bis 7, **dadurch gekennzeichnet, dass** der Lichtleiter (2) - endseitig - mit der Licht emittierenden Fläche (4) bündig verklebt ist.

9. Handgerät nach Anspruch 8, **dadurch gekennzeichnet, dass** der Lichtleiter (2) mittels transparentem Klebstoff (5) mit der Licht emittierenden Fläche (4) verklebt ist.

10. Handgerät nach Anspruch 8 oder 9, **dadurch gekennzeichnet, dass** der Klebstoff (5) die Licht emittierende Fläche (4) insgesamt im Sinne einer Abdeckmaske - anstelle eines Linsenkörpers - überdeckt.

11. Handgerät nach Anspruch 10, **dadurch gekennzeichnet, dass** der über die Querschnittsfläche des Lichtleiters (2) hinausragende Bereich der Abdeckmaske zumindest weitgehend lichtundurchlässig ist.

12. Handgerät nach einem der Ansprüche 8 bis 11, **dadurch gekennzeichnet, dass** die Licht emittierenden Fläche (4) in etwa dem Faserquerschnitt angepasst ist.

13. Handgerät nach einem der Ansprüche 1 bis 12, **dadurch gekennzeichnet, dass** der Verbindungsbereich zwischen Lichtleitfaser (2) und Licht emittierender Fläche (4) optisch und/oder mechanisch versiegelt ist.

## Claims

1. Handheld medical device for illumination, in particular for use in ophthalmology, having a housing serving as a handle, a probe (1) extending from the housing with an optical waveguide (2) running therein and a light outlet at the end, and a light source (3) integrated into the housing,
wherein the light source (3) is in the form of an LED light source with a light-emitting surface (4), wherein the optical waveguide (2) is directly adjacent to the light-emitting surface (4), and wherein the optical waveguide (2) is bonded at the end to the light-emitting surface (4),
**characterised in that** the region of the light-emitting surface that projects beyond the cross-sectional area of the optical waveguide (2) is covered by a mask that is impervious to light, so that all the light emitted from the surface is coupled into the optical waveguide.

2. Handheld device according to claim 1, **characterised in that** the light source (3) is in the form of a light-emitting chip.

3. Handheld device according to claim 1 or 2, **characterised in that** the optical waveguide (2) is in the form of an optical fibre, preferably in the form of a glass fibre.

4. Handheld device according to claim 3, **characterised in that** the optical waveguide (2) is in the form of a monofibre.

5. Handheld device according to claim 3, **characterised in that** the optical waveguide (2) is in the form of a fibre bundle.

6. Handheld device according to any one of claims 1 to 5, **characterised in that** the optical waveguide (2) is oriented approximately orthogonally to the light-emitting surface (4).

7. Handheld device according to any one of claims 1 to 6, **characterised in that** the optical waveguide (2) abuts the light-emitting surface (4) with at least one planar surface.

8. Handheld device according to any one of claims 1 to 7, **characterised in that** the optical waveguide (2) - at the end - is bonded flush to the light-emitting surface (4).

9. Handheld device according to claim 8, **characterised in that** the optical waveguide (2) is bonded to the light-emitting surface (4) by means of transparent adhesive (5).

10. Handheld device according to claim 8 or 9, **characterised in that** the adhesive (5) covers the light-emitting surface (4) completely in the manner of a mask - instead of a lens body.

11. Handheld device according to claim 10, **characterised in that** the region of the mask that projects beyond the cross-sectional area of the optical waveguide (2) is at least largely impervious to light.

12. Handheld device according to any one of claims 8 to 11, **characterised in that** the light-emitting surface (4) is adapted approximately to the fibre cross-section.

13. Handheld device according to any one of claims 1 to 12, **characterised in that** the bonding region between the optical fibre (2) and the light-emitting surface (4) is sealed optically and/or mechanically.

## Revendications

1. Pièce à main médicale pour éclairage, en particulier destinée à être utilisée en ophtalmologie, comportant un boîtier faisant fonction de poignée, une sonde (1), qui s'étend à partir du boîtier et est munie d'un guide de lumière (2) s'étendant à l'intérieur de celle-ci et d'une sortie de lumière à une extrémité, et une source lumineuse (3) intégrée dans le boîtier,
ladite source lumineuse (3) étant réalisée sous la forme d'un source lumineuse à diodes électroluminescentes avec une surface (4) émettant de la lumière, ledit guide de lumière (2) étant directement adjacent à la surface (4) émettant de la lumière, et ledit guide de lumière (2) étant collé par une extrémité contre la surface (4) émettant de la lumière,
**caractérisée en ce que** la zone de la surface émettant de la lumière, laquelle zone s'avance au-delà de la surface transversale du guide de lumière (2), est recouverte par un masque de recouvrement opaque, de telle sorte que la lumière émise hors de la surface est injectée intégralement dans le guide de lumière.

2. Pièce à main selon la revendication 1, **caractérisée en ce que** la source lumineuse (3) est réalisée sous la forme d'une pastille lumineuse.

3. Pièce à main selon la revendication 1 ou 2, **caractérisée en ce que** le guide de lumière (2) est réalisé sous la forme d'une fibre optique, de préférence sous forme de fibre de verre.

4. Pièce à main selon la revendication 3, **caractérisée en ce que** le guide de lumière (2) est réalisé sous la forme d'une monofibre.

5. Pièce à main selon la revendication 3, **caractérisée en ce que** le guide de lumière (2) est réalisé sous la forme d'un faisceau de fibres.

6. Pièce à main selon l'une quelconque des revendications 1 à 5, **caractérisée en ce que** le guide de lumière (2) est aligné sensiblement orthogonalement à la surface (4) émettant de la lumière.

7. Pièce à main selon l'une quelconque des revendications 1 à 6, **caractérisée en ce que** le guide de lumière (2) est adjacent, par au moins une surface plane, à la surface (4) émettant de la lumière.

8. Pièce à main selon l'une quelconque des revendications 1 à 7, **caractérisée en ce que** le guide de lumière (2) - par une extrémité-est collé à fleur de la surface (4) émettant de la lumière.

9. Pièce à main selon la revendication 8, **caractérisée en ce que** le guide de lumière (2) est collé à la surface (4) émettant de la lumière au moyen d'une colle (5) transparente.

10. Pièce à main selon la revendication 8 ou 9, **caractérisée en ce que** la colle (5) couvre la surface (4) émettant de la lumière dans son ensemble dans le sens d'un masque de recouvrement - en lieu et place d'un corps de lentille.

11. Pièce à main selon la revendication 10, **caractérisée en ce que** la zone du masque de recouvrement, laquelle s'avance au-delà de la surface transversale du guide de lumière (2), est au moins en grande partie opaque.

12. Pièce à main selon l'une quelconque des revendications 8 à 11, **caractérisée en ce que** la surface (4) émettant de la lumière est ajustée sensiblement à la section transversale d'une fibre.

13. Pièce à main selon l'une quelconque des revendications 1 à 12, **caractérisée en ce que** la zone de liaison entre la fibre du guide de lumière (2) et la surface (4) émettant de la lumière est scellée optiquement et/ou mécaniquement.
